# EUROPÄISCHE PATENTSCHRIFT

(11) **EP 2 192 891 B1**
(45) Veröffentlichungstag und Bekanntmachung des Hinweises auf die Patenterteilung: **16.03.2011**
(21) Anmeldenummer: 08838505.9
(22) Anmeldetag: 02.10.2008
(51) Int. Cl.: A61K 8/97, A61K 8/67, A61Q 11/00

(54) **STOFFZUSAMMENSETZUNG FÜR MUNDHYGIENEPRODUKT**
MATERIAL COMPOSITION FOR ORAL HYGIENE PRODUCT
COMPOSITION POUR PRODUIT D'HYGIÈNE BUCCALE

(30) Priorität: 02.10.2007 DE 102007047237
(43) Veröffentlichungstag der Anmeldung: 09.06.2010
(73) Patentinhaber: Busch, Klaus-Uwe, 5600 St. Johann im Pongau (AT)
(72) Erfinder: Busch, Klaus-Uwe, 5600 St. Johann im Pongau (AT)
(74) Vertreter: Heselberger, Johannes
(86) Internationale Anmeldenummer: PCT/EP2008/008386
(87) Internationale Veröffentlichungsnummer: WO 2009/046942

(56) Entgegenhaltungen:
- EP-A- 0 311 259
- WO-A-00/67711
- WO-A-03/097000
- WO-A1-2005/063184
- US-A- 4 272 512

## Beschreibung

### 1. Gebiet der Erfindung

Die vorliegende Erfindung bezieht sich auf eine Stoffzusammensetzung, insbesondere für ein Mundhygieneprodukt, wie Zahnpasten, Mundspülungen, Zahnseiden und Zahnreinigungskaugummis. Die Stoffzusammensetzung soll insbesondere zur Bekämpfung von Karies, Paradontitis und Gingivitis geeignet sein.

### 2. Stand der Technik

Mundhygieneprodukte werden in vielfältiger Weise zur Vorbeugung gegen und zur Behandlung von Krankheiten der Zähne und / oder des Mundraums eingesetzt. Ein Mundhygieneprodukt kann beispielsweise eine Zahnpasta, eine Mundspülung, ein Spray, eine Zahnseide, ein Kaugummi, ein Zungenreiniger oder ein Nahrungsergänzungsmittel sein. Je nach Anwendung enthält das Mundhygieneprodukt verschiedene Wirkstoffe.

In vielen konventionellen Zahnpasten und anderen Mundhygieneprodukten wird als Wirkstoff gegen Karies Fluorid eingesetzt. Fluorid härtet den Zahnschmelz und verhindert dadurch das Eindringen von Bakterien in den Zahn. Durch den Einsatz eines Extrakts aus der Pflanze Curcuma Xanthorrhiza eröffnet sich die Möglichkeit, Karies auf eine völlig andere Art zu bekämpfen, indem die Anzahl der kariesverursachenden Bakterien in der Mundhöhle stark reduziert wird. Im US-Patent 6,696,404 wird eine antibakterielle Zusammensetzung offenbart, die den Extrakt aus C.X. enthält und als Mundhygieneprodukt eingesetzt werden kann.

Der Extrakt aus der Curcuma Xanthorrhiza (javanische Gelbwurz) wird seit langem als natürliches Heilmittel bei Leber- und Gallenbeschwerden eingesetzt. Der Extrakt aus Curcuma Xanthorrhiza (im Folgenden als C.X.-Extrakt abgekürzt) wirkt entzündungshemmend und antibakteriell. Insbesondere kann C.X.-Extrakt auch gegen Bakterien wie Streptococcus mutans eingesetzt werden, welche in der Mundhöhle vorkommen und für Karies ursächlich sind. C.X.-Extrakt ist als Nahrungsmittel zugelassen. Die Einnahme dieses Extrakts ist also auch in höheren Konzentrationen gesundheitlich unbedenklich.

Eine andere Mundkrankheit ist Parodontitis (oft fälschlich Parodontose genannt), welche eine Entzündung des Zahnhalteapparats (Kieferknochen, Zahnfleisch, Zahnhals, Wurzelzement und Wurzelhaut) beschreibt. Für Parodontitis sind Zahnbeläge (Plaque), welche Bakterien enthalten, verantwortlich. Ein anderes, aber ähnliches Krankheitsbild stellt die Gingivitis, eine Entzündung des Zahnfleisches, dar. Im US-Patent 4,272,512 wird eine Zusammensetzung offenbart, die unter anderem Folsäure enthält und zur Therapie von Gingivitissymptomen wie entzündetem Zahnfleisch eingesetzt wird. In diesem Dokument des Stands der Technik wird die Folsäure insbesondere in Verbindung mit Tranexamsäure verwendet.

Folsäure ist ein Vitamin aus dem B-Komplex, weshalb es auch als Vitamin B9 bezeichnet wird. Folsäure wirkt reiz- und entzündungshemmend. Weiterhin beeinflusst Folsäure die Immunabwehr positiv, was eine gesunde Mundschleimhaut fördert. Während der Schwangerschaft ist eine ausreichende Folsäureversorgung zur Vermeidung der Häufigkeit von Fehlbildungen des Fötus (insbesondere der Neuralrohrdefekt) von Bedeutung.

WO-00/67711 und WO-03/097000 offenbaren antibakterielle Zubereitungen zur Verbesserung der Mundhygiene, die Xanthorrhizol oder einen Extrakt aus Curcuma Xanthorrhiza enthalten.

In der internationalen Patentanmeldung WO 2005/063184 A1 wird eine Stoffzusammensetzung offenbart, die bei der Mundhygiene von Haustieren zum Einsatz kommen soll (vgl. Seite 2, Zeilen 6 - 17 des Dokuments). Die Bestandteile dieser Stoffzusammensetzung sind hauptsächlich Xylitol, ein Enzym mit dem Namen "Emilgase", ein Konservierungsmittel und ein antimikrobieller Wirkstoff. Als weitere mögliche Bestandteile der Stoffzusammensetzung werden unter anderem Xanthorrhizol und Folsäure erwähnt.

Die vorliegende Erfindung hat sich die Aufgabe gestellt, eine Stoffzusammensetzung bzw. ein Mundhygieneprodukt mit einer erhöhten Wirksamkeit bezüglich der Vorbeugung bzw. Behandlung von Karies, Parodontitis und Gingivitis bereit zu stellen.

### 3. Zusammenfassung der Erfindung

Diese Aufgabe wird durch eine Stoffzusammensetzung nach Patentanspruch 1 gelöst, welche sowohl einen Extrakt aus C.X. als auch Folsäure aufweist. Der Extrakt aus C.X. besteht aus Xanthorrizol und weiteren pflanzlichen Verbindungen. Im Rahmen der vorliegenden Erfindung kommt es nicht darauf an, mit welchem Verfahren der Extrakt aus C.X. gewonnen wurde. Die Verwendung eines Kohlenstoffdioxid-Extraktionsverfahrens bietet in diesem Zusammenhang Vorteile, insbesondere weil dieses lösungsmittelfrei arbeitet.

Ferner wurde festgestellt, dass die Verwendung von Xanthorrhizol (hydroxy ar(omatisch)-curcumen), einem Bestandteil von C.X., statt eines Extrakts aus C.X., das neben Xanthorrhizol typischerweise noch ar(omatisch)-curcumen und beta-curcumen aufweist, zu den erfindungsgemäßen Vorteilen führt. Xanthorrhizol macht typischerweise rund 25 Gew.-% des Xanthorrhiza-Öls aus.

Bei der Kombination der beiden Stoffe wird ein überraschender synergetischer Effekt erzielt. Wie eine, unten ausführlicher dargestellte medizinische Studie der Anmelderin belegt, führt die Kombination des Extrakts aus C.X. bzw. Xanthorrhizol und Folsäure in einer Zahnpasta zu einer deutlichen Senkung der Erkrankungsrisiken für die drei Zahnkrankheiten Karies, Parodontitis und Gingivitis. Insbesondere werden durch eine derartige Zahnpasta die Erkrankungsrisiken bezüglich allen drei Zahnkrankheiten (Karies, Parodontitis, Gingivitis) um 80 bis 90 Prozent reduziert. Diese hohe Wirksamkeit der Kombination kann nur dadurch erklärt werden, dass sich die beiden Wirkstoffe (der Extrakt aus C.X. bzw. Xanthorrhizol und Folsäure) in ihrer Wirkung gegenseitig verstärken. Dies war bislang unbekannt.

Erfindungsgemäß beträgt die Konzentration der Folsäure in der Stoffzusammensetzung zwischen 0,02 Gew.-% und 0,2 Gew.-%. Die Konzentration des Extrakts von C.X. in der Stoffzusammensetzung beträgt erfindungsgemäß mindestens 0,005 Gew.-%, besonders bevorzugt mindestens 0,01 Gew.-% und weiter bevorzugt mindestens 0,03 Gew.-%. Falls statt eines Extrakts von C.X. Xanthorrhizol verwendet wird, sollte dessen Konzentration in der Stoffzusammensetzung erfindungsgemäß mindestens 0,0015 Gew.-% betragen, besonders bevorzugt mindestens 0,003 Gew.-% und weiter bevorzugt mindestens 0,01 Gew.-%.

In einer bevorzugten Ausführungsform der Erfindung weist die Stoffzusammensetzung zusätzlich die Vitamine B6 und B12 auf. Die Zugabe der Vitamine B6 und B12 steigert die Wirksamkeit des Mundhygieneprodukts gegenüber Parodontitis und Gingivitis noch einmal.

Weiter vorzugsweise enthält die Stoffzusammensetzung kein Fluorid, im Gegensatz zu traditionellen Stoffzusammensetzungen zur Vorbeugung gegen Karies. Die hohe Wirksamkeit der erfindungsgemäßen Stoffzusammensetzung ist die Ursache, dass in einer bevorzugten Ausführungsform der Erfindung auf die Zugabe von Fluorid verzichtet werden kann.

Aufgrund seiner guten Verträglichkeit und hohen Wirksamkeit kann ein Mundpflegeprodukt gemäß der Erfindung besonders auch während der Schwangerschaft verwendet werden.

Die erfindungsgemäße Stoffzusammensetzung eignet sich für die Herstellung eines Mundhygieneproduktes, und kann insbesondere als Bestandteil - neben den jeweiligen üblichen Bestandteilen - einer Zahnpasta, eines Mundsprays, einer Mundspülung, einer Zahnseide, eines Zahnreinigungskaugummis oder eines Nahrungsergänzungsmittels eingesetzt werden.

### 4. Detaillierte Beschreibung der Erfindung

Die Anmelderin führte eine klinische Studie mit achtjähriger Laufzeit durch, in der der Effekt verschiedener Zahnpasten auf die Häufigkeit der Zahnkrankheiten Karies, Paradontitis und Gingivitis untersucht wurde, wobei die verschiedenen Zahnpasten jeweils andere Wirksubstanzen oder Kombinationen derselben enthielten.

In dieser medizinischen Studie gab es insgesamt 9 Gruppen, davon 3 Gruppen mit Schwangeren. Die anderen 6 Gruppen bestanden jeweils zu Hälfte aus Männern und Frauen. Weiterhin wurden die Probanden so ausgesucht und eingeteilt, dass sich gleichwertige Testgruppen ergaben bezüglich des Mundzustands der Gruppenmitglieder bei Beginn der Studie, der allgemeinen Gesundheit der Gruppenmitglieder, des Alters der Gruppenmitglieder sowie der mentalen Fähigkeiten der Gruppenmitglieder, eine mindestens durchschnittliche Mundhygiene anwenden zu können.

Die Mundhygiene der Teilnehmer an der Studie wurde durch ein intensives Mundhygienetraining standardisiert. Vor Beginn der Studie bekam jeder Proband eine PZR-Programm (professionelle Zahnreinigung) und bis auf die Zahnpasta gleiches Mundhygienematerial (Zahnbürste).

Alle Testgruppen erhielten dieselbe Basiszahnpasta. Die Basiszahnpasta der Studie bestand aus Wasser, Feuchthaltemitteln, einem Putzkörper, Aroma, Süßstoff (Saccharin) und Fluorid. Nur in einer der Testgruppen enthielt die Basiszahnpasta kein Fluorid (siehe Gruppe 9 unten). Die Zahnpasten der einzelnen Testgruppen unterscheiden sich dadurch, dass der Basiszahnpasta zusätzliche Wirkstoffe beigegeben wurden:

| | |
|---|---|
| Gruppe 1: | nur Basiszahnpasta (Kontrollgruppe). |
| Gruppe 2: | Zusatz von C.X.-Extrakt und Folsäure zur Basiszahnpasta. |
| Gruppe 3: | Zusatz von C.X-Extrakt zur Basiszahnpasta. |
| Gruppe 4: | Zusatz von Folsäure zur Basiszahnpasta. |
| Gruppe 5: | Zusatz von C.X.-Extrakt, Folsäure, B6 und B 12 zur Basiszahnpasta. |
| Gruppe 6: | Zusatz von B6 und B 12 zur Basiszahnpasta. |
| Gruppe 7: | Schwangere mit Basiszahnpasta. |
| Gruppe 8: | Schwangere mit Zusatz von C.X.-Extrakt und Folsäure zur Basiszahnpasta. |
| Gruppe 9: | Schwangere mit Zusatz von C.X.-Extrakt und Folsäure zur Basiszahnpasta ohne Fluor. |

Dabei wurden den entsprechenden Zahnpasten die folgenden Konzentrationen der Wirkstoffe beigegeben: C.X.-Extrakt: 0,03 Gew.% (der Wirkstoff wurde aus der biologisch angebauten Pflanzen durch CO₂-Extraktion extrahiert);
Folsäure: 0,08 Gew.%
Vitamin B6: 0,01 Gew.% und
Vitamin B12: 0,002 Gew.%

Es versteht sich, dass aber auch Zahnpasten mit anderen Konzentrationen an Folsäure, C.X.-Extrakt, B6 und / oder B 12 unter bestimmten Umständen vorteilhaft sind und demnach zum Gegenstand der Erfindung gehören.

Das Kariesrisiko wurde vermessen, indem die Zahl der neu auftretenden kariösen Zähne in Relation zur Kontrollgruppe 1 bzw. 7 (bei Schwangeren) gemessen wurde, wobei die Kontrollgruppen lediglich die Basiszahnpasta anwendete. Die Messungen von neu auftretenden kariösen Zähnen bei den Gruppen 1 und 7 wurden als Referenzmaß (100%) angenommen.

Ähnlich wurde die auftretende Gingivitis und Paradontitis der einzelnen Testgruppen in Relation zur Kontrollgruppe 1 bzw. 7 (bei Schwangeren) vermessen. Die Messungen bei den Gruppen 1 und 7 wurden wieder als 100% angenommen.

Alle Probanden der Gruppen 1 - 6 wurden einmal pro Quartal, also viermal jährlich, zur Auswertung untersucht. Die Gruppen 7 - 9 der Schwangeren wurden monatlich untersucht. Alle oben beschriebenen Messmethoden zu den Zahnkrankheiten wurden standardisiert und über die gesamte Zeit der Studie nicht verändert. Als Ergebnis der Studie zu den drei Zahnkrankheiten ergab sich im Einzelnen:

### Kariesrisiko:

Eine Zahnpasta mit Zusatz von C.X.-Extrakt allein konnte das Kariesrisiko um 20-25% reduzieren (Gruppe 3). Dagegen hatte eine Zahnpasta mit Folsäure allein keinen Effekt auf das Kariesrisiko (Gruppe 4). Die Zugabe von C.X.-Extrakt und Folsäure in Kombination reduzierte das Kariesrisiko um bis zu 80% (Gruppen 2, 8 und 9). Damit zeigt sich ein deutlicher synergetischer Effekt der Kombination C.X.-Extrakt und Folsäure, der weit über den Effekt von C.X.-Extrakt allein hinausgeht.

Außerdem ist es bemerkenswert, dass bei der Gruppe 9 der Schwangeren, die eine Zahnpasta ohne Fluorid benutzte, das Ergebnis für das Kariesrisiko gleich gut ist. Dies legt den Schluss nahe, dass auf die Zugabe von Fluorid bei Schwangeren verzichtet werden kann, ohne ihr Kariesrisiko zu erhöhen.

Die Zugabe von Vitaminen B6 und B 12 zusätzlich zu C.X.-Extrakt und Folsäure hatten keinen Effekt auf das Kariesrisiko (Gruppe 5), wie auch die Zugabe der Vitamine B6 und B 12 allein keinen Effekt auf das Kariesrisiko hatte (Gruppe 6).

### Gingivitisrisiko:

Eine Zahnpasta mit C.X.-Extrakt allein konnte das Gingivitisrisiko um 15% reduzieren (Gruppe 3). Eine Zahnpasta mit Folsäure allein konnte das Gingivitisrisiko um bis zu 30% reduzieren (Gruppe 4). Dagegen reduziert eine Zahnpasta mit C.X.-Extrakt und Folsäure in Kombination das Gingivitisrisiko um bis zu 82% (Gruppe 2). Diese Reduktion um 82% ist deutlich mehr als die Summe der Einzelwirkungen von 15% und 30%.

Für die Schwangeren in den Gruppen 8 und 9 konnte durch die Kombination von C.X.-Extrakt und Folsäure sogar eine Reduzierung des Gingivitisrisikos um über 95% gemessen werden.

Die Zugabe von Vitaminen B6 und B12 zusätzlich zu C.X.-Extrakt und Folsäure konnte das Gingivitisrisiko um bis zu 91% bei den männlichen Probanden reduzieren (männlicher Teil der Gruppe 5), während bei den Probandinnen die Zugabe der Vitamine keinen zusätzlichen Effekt hatte (weiblicher Teil der Gruppe 5). Gleichfalls hatte eine Zahnpasta mit alleiniger Zugabe von Vitamin B6 und B12 keinen nennenswerten Effekt auf das Gingivitisrisiko (Gruppe 6).

### Parodontitisrisiko:

Die Zahnpasta mit Zugabe von C.X.-Extrakt allein konnte das Parodontitisrisiko um 18% reduzieren (Gruppe 3), während die Zugabe von Folsäure allein das Parodontitisrisiko um bis zu 35% reduzieren konnte (Gruppe 4). Dagegen reduzierte eine Zahnpasta mit C.X.-Extrakt und Folsäure in Kombination das Parodontitisrisiko um bis zu 79% (Gruppe 2), was wieder deutlich mehr als die Addition von 18% und 35% ist.

Bei den Gruppen 8 und 9 der Schwangeren erreichte eine Zahnpasta, die die Kombination von C.X.-Extrakt und Folsäure enthielt, sogar eine Reduzierung des Paradontitisrisikos um über 90%.

Wie bei der Gingivitis konnte die Zugabe der Vitamine B6 und B12 zusätzlich zu C.X.-Extrakt und Folsäure das Parodontitisrisiko um bis zu 91 % (gegenüber 79% bei der Kombination von C.X.-Extrakt und Folsäure) bei den männlichen Probanten reduzieren (männlicher Teil der Gruppe 5), während bei den weiblichen Probandinnen die Zugabe wieder keinen zusätzlichen Effekt zeigte (weiblicher Teil der Gruppe 5). Auch hatte eine Zahnpasta mit der Zugabe der Vitamine B6 und B 12 keinen nennenswerten Effekt auf das Parodontitisrisiko (Gruppe 6).

### Schlussfolgerungen:

Insgesamt konnte eine Zahnpasta mit der Kombination von C.X.-Extrakt und Folsäure die Erkrankungshäufigkeit bei allen drei Zahnkrankheiten deutlich herabsetzen (zwischen 80 und 90%). Diese große Reduzierung des Erkrankungsrisikos war überraschend, da die beiden Wirkstoffe C.X.-Extrakt und Folsäure für sich alleine bei weitem nicht so stark wirken und ein synergetischer Effekt nicht zu erwarten war. Die Studie belegt dagegen einen synergetischen Effekt von C.X-Extrakt und Folsäure auf die Mundgesundheit. Für Schwangere ergibt sich als besonderer Effekt, dass die Kombination von C.X.-Extrakt und Folsäure den Verzicht auf Fluorid möglich macht, ohne dass sich der Kariesschutz verschlechtert. Der Zusatz der Vitamine B6 und B12 zusätzlich zu C.X.-Extrakt und Folsäure verringert das Erkrankungsrisiko an Gingivitis und Paradontitis weiter, allerdings nur bei männlichen Patienten.

Obwohl in der hier beschriebenen Studie die Kombination von C.X.-Extrakt und Folsäure in einer Zahnpasta eingesetzt wurde, ist der Einsatz dieser Wirkstoff kombination in einer Mundspülung, einer Zahnseide, einem Kaugummi, einem Mundspray, einem Zungenreinigungsprodukt oder einem Nahrungsergänzungsmittel ebenfalls vorgesehen, da hier ähnliche Synergieeffekte zwischen C.X.-Extrakt und Folsäure erwartet werden können.

## Patentansprüche

1. Eine Stoffzusammensetzung, die
a. einen Extrakt aus Curcuma Xanthorrhiza; und
b. den Wirkstoff Folsäure
enthält,
**dadurch gekennzeichnet, dass** die Konzentration der Folsäure zwischen 0,02 Gew.-% und 0,2 Gew.-% beträgt und
die Konzentration des Extrakts aus Curcuma Xanthorrhiza mindestens 0,005 Gew.-% beträgt.

2. Eine Stoffzusammensetzung, die
a. Xanthorrhizol; und
b. den Wirkstoff Folsäure
enthält,
**dadurch gekennzeichnet, dass** die Konzentration der Folsäure zwischen 0,02 Gew.-% und 0,2 Gew.-% beträgt und
die Konzentration des Xanthorrhizols mindestens 0,0015 Gew.-% beträgt.

3. Stoffzusammensetzung gemäß Anspruch 1 und 2, **dadurch gekennzeichnet, dass** sie zusätzlich die Vitamine B6 und B 12 enthält.

4. Stoffzusammensetzung gemäß einem der vorhergehenden Ansprüche, **dadurch gekennzeichnet, dass** sie kein Fluorid enthält.

5. Mundhygieneprodukt, **dadurch gekennzeichnet, dass** es eine Stoffzusammensetzung nach einem der Ansprüche 1 bis 4 umfasst.

6. Zahnpasta, **dadurch gekennzeichnet, dass** es eine Stoffzusammensetzung nach einem der Ansprüche 1 bis 4 umfasst.

7. Mundspülung, **dadurch gekennzeichnet, dass** es eine Stoffzusammensetzung nach einem der Ansprüche 1 bis 4 umfasst.

8. Zahnseide, **dadurch gekennzeichnet, dass** es eine Stoffzusammensetzung nach einem der Ansprüche 1 bis 4 umfasst.

9. Zahnreinigungskaugummi, **dadurch gekennzeichnet, dass** es eine Stoffzusammensetzung nach einem der Ansprüche 1 bis 4 umfasst.

10. Zungenreinigungsprodukt, **dadurch gekennzeichnet, dass** es eine Stoffzusammensetzung nach einem der Ansprüche 1 bis 4 umfasst.

11. Nahrungsergänzungsmittel, **dadurch gekennzeichnet, dass** es eine Stoffzusammensetzung nach einem der Ansprüche 1 bis 4 umfasst.

12. Stoffzusammensetzung gemäß einem der Ansprüche 1 bis 4 zur Behandlung von Karies.

13. Stoffzusammensetzung gemäß einem der Ansprüche 1 bis 4 zur Behandlung von Gingivitis.

14. Stoffzusammensetzung gemäß einem der Ansprüche 1 bis 4 zur Behandlung von Paradontitis.

15. Stoffzusammensetzung gemäß einem der Ansprüche 1 bis 4 zur Gewährleistung einer ausreichenden Folsäureversorgung während der Schwangerschaft.

## Claims

1. A material composition which comprises
a. an extract from Curcuma Xanthorrhiza; and
b. the substance folic acid,
**characterized in that** the concentration of folic acid is between 0.02 weight % and 0.2 weight % and
the concentration of Curcuma Xanthorrhiza is at least 0.005 weight %.

2. A material composition which comprises
a. Xanthorrhizol; and
b. the substance folic acid,
**characterized in that** the concentration of folic acid is between 0.02 weight % and 0.2 weight % and
the concentration of Xanthorrhizol is at least 0.0015 weight %.

3. Material composition according to claim 1 or 2, **characterized in that** it additionally comprises the vitamins B6 and B12.

4. Material composition according to any of the preceding claims, **characterized in that** it does not comprise any fluoride.

5. Oral hygiene product, **characterized** it that it comprises a material composition according to any of claims 1 to 4.

6. Toothpaste, **characterized in that** it comprises a material composition according to any of claims 1 to 4.

7. Oral rinse, **characterized** it that it comprises a material composition according to any of claims 1 to 4.

8. Dental floss, **characterized in that** it comprises a material composition according to any of claims 1 to 4.

9. Chewing gum for dental cleaning, **characterized** it that it comprises a material composition according to any of claims 1 to 4.

10. Tongue cleaner, **characterized in that** it comprises a material composition according to any of claims 1 to 4.

11. Food supplement, **characterized in that** it comprises a material composition according to any of claims 1 to 4.

12. Material composition according to any of claims 1 to 4 for the treatment of caries.

13. Material composition according to any of claims 1 to 4 for the treatment of gingivitis.

14. Material composition according to any of claims 1 to 4 for the treatment of parodontitis.

15. Material composition according to any of claims 1 to 4 for ensuring a sufficient supply of folic acid during pregnancy.

## Revendications

1. Composition de matière, qui contient
a. un extrait de Curcuma xanthorrhiza ; et
b. la matière active acide folique,
**caractérisée en ce que** la concentration de l'acide folique est comprise entre 0,02 et 0,2 % en poids, et que la concentration de l'extrait de Curcuma xanthorrhiza est d'au moins 0,005 % en poids.

2. Composition de matière, qui contient
a. du xanthorrhizol ; et
b. la matière active acide folique,
**caractérisée en ce que** la concentration de l'acide folique est comprise entre 0,02 et 0,2 % en poids, et que la concentration du xanthorrhizol est d'au moins 0,0015 % en poids.

3. Composition de matière selon la revendication 1 ou 2, **caractérisée en ce qu'**elle contient en outre les vitamines B6 et B12.

4. Composition de matière selon l'une des revendications précédentes, **caractérisée en ce qu'**elle ne contient aucun fluorure.

5. Produit pour l'hygiène buccale, **caractérisé en ce qu'**il comprend une composition de matière selon l'une des revendications 1 à 4.

6. Pâte dentaire, **caractérisée en ce qu'**elle comprend une composition de matière selon l'une des revendications 1 à 4.

7. Bain de bouche, **caractérisé en ce qu'**il comprend une composition de matière selon l'une des revendications 1 à 4.

8. Fil dentaire, **caractérisé en ce qu'**il comprend une composition de matière selon l'une des revendications 1 à 1.

9. Gomme à mâcher pour le nettoyage des dents, **caractérisée en ce qu'**elle comprend une composition de matière selon l'une des revendications 1 à 14.

10. Produit pour le nettoyage des dents, **caractérisé en ce qu'**il comprend une composition de matière selon l'une des revendications 1 à 4.

11. Produit complément alimentaire, **caractérisé en ce qu'**il comprend une composition de matière selon l'une des revendications 1 à 4.

12. Composition de matière selon l'une des revendications 1 à 4 pour le traitement des caries.

13. Composition de matière selon l'une des revendications 1 à 4 pour le traitement de la gingivite.

14. Composition de matière selon l'une des revendications 1 à 4 pour le traitement de la parodontite.

15. Composition de matière selon l'une des revendications 1 à 4 pour assurer un apport suffisant en acide folique pendant la grossesse.
